# EUROPEAN PATENT SPECIFICATION

(11) **EP 1 490 132 B1**
(45) Date of publication and mention of the grant of the patent: **16.07.2008**
(21) Application number: 03711860.1
(22) Date of filing: 26.03.2003
(51) Int. Cl.: A61M 5/168, G05B 9/03

(54) **A SAFETY SYSTEM FOR AN ELECTRICALLY DRIVEN MEDICAL DELIVERY DEVICE AND A COMPUTER-READABLE MEDIUM**
SICHERHEITSSYSTEM FÜR EIN ELEKTRISCH BETRIEBENES MEDIZINISCHES VERABREICHUNGSSYSTEM UND RECHNERLESBARES MEDIUM
SYSTEME DE SECURITE POUR DISPOSITIF D'ADMINISTRATION MEDICAL A COMMANDE ELECTRIQUE, ET SUPPORT LISIBLE PAR ORDINATEUR

(30) Priority: 27.03.2002 DK 200200468
(43) Date of publication of application: 29.12.2004
(73) Proprietor: NOVO NORDISK A/S, 2880 Bagsvaerd (DK)
(72) Inventor: Jespersen, Soren Kragh, 3480 Fredensborg (DK); Munk, Jens Aage, 3650 Olstykke (DK); Radmer, Jim, 3480 Fredensborg (DK); Poulsen, Jens Ulrik, 2830 Virum (DK); CHRISTENSEN, Lars, Hofmann, DK-4040 Jyllinge (DK); KLITMOSE, Lars, Peter, DK-2820 Gentofte (DK)
(74) Representative: Kellberg, Lars
(86) International application number: PCT/DK2003/000205
(87) International publication number: WO 2003/080159

(56) References cited:
- EP-A- 0 028 364
- EP-A- 0 118 008
- EP-A- 0 384 155
- EP-A- 0 800 979
- US-A- 4 897 184
- US-A- 5 532 562

## Description

The invention relates to a safety system for an electrically driven medical delivery device for delivering a dose of a medication, wherein the safety system is configured for preventing delivery of an erroneous dose of the medicament and comprising detector means for registering mechanical movement. The closest prior art is EP-A- 0118008, which defines the preambles of claims 1 and 15.

Systems of that kind are known e.g. from US 6,259,587, which teaches the use of microprocessors having feedback from a detector means for increasing the safety against erroneous dose.

US 4,722,734 comprises the use of a tachometer, and it also mentions the option of increasing the safety by building a time limitation into the safety system.

EP 384 155 comprises a pair of redundant control circuits comprising sensors for detecting whether the delivery device is active.

Developments within pumps and the like equipment for dispensing a dose of medication have become increasingly sophisticated such that, today, doses can be dispensed relatively expediently and, besides, increasingly concentrated medicaments are used, which means that the systems known so far have not provided a sufficient degree of safety against overdoses, due to the prior art systems having more or less presupposed that the dose was administered over such protracted period of time that an upper time limit was sufficient to effectively ensure that an overdose was not delivered.

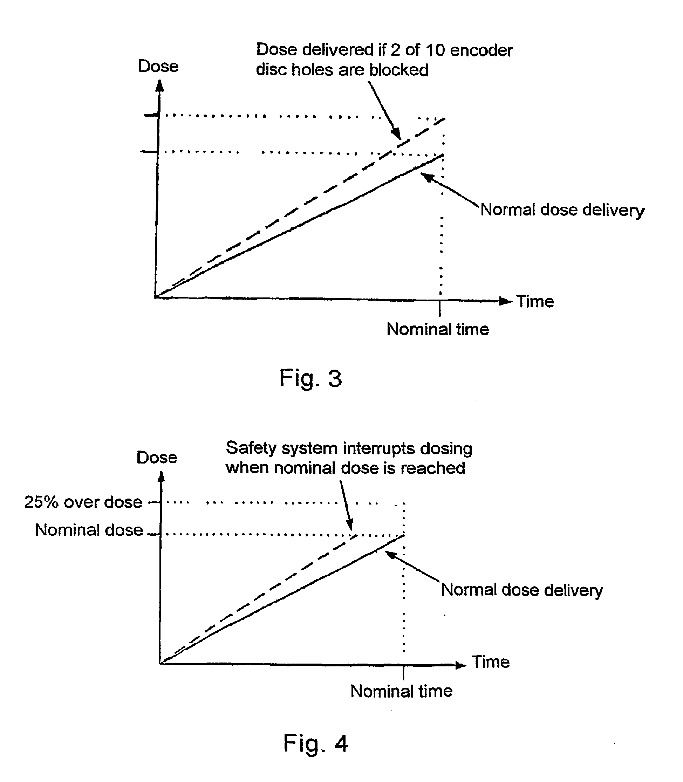

This is demonstrated with reference to fig. 3 in the drawing. The full line shows the delivery of a normal dose within a normal time and the dashed line shows 25% overdose within the normal time, if - it happens - two out of ten disk holes in a commonly used detector means are blocked. An overdose of 25% of the most modern insulin types may be lethal.

It is the object of the invention to provide a system that works very quickly, accurately and reliably, i.e. reacts very swiftly in case conditions are detected that seem to point to an overdose. This object may be provided without a limit of time and further even though one of the safety control circuits may fail.

This object is achieved by the invention defined in claim 1, which describes a safety system comprising at least two control circuits configured for controlling the power supply to driving means for the delivery device; and wherein at least two detector means are provided each being arranged for detecting a mechanical parameter for delivering the dose and for emitting a signal to a respective one of said control circuits.

The high demands made today to the accuracy of delivery of dose and to a safety system being able to expediently preventing an overdose are fulfilled in that both safety circuits receive information from separate, respective detector means, said information being based on a reliable measurement of the mechanical movements that operate the medical delivery device. Such advantageous properties are obtained no matter how the safety circuits are configured, thus they may be a master and slave circuit or the control circuit may comprise two separately operating control circuits.

A further advantage obtained by two separate detector means is that the signals emitted from the detector means may be processed in the control circuits. Thereby it is possible to take into account any deviation from normal function, e.g. in case of a mismatch a dedicated action can be made in the control circuits.

A preferred mechanical parameter is a rotational movement that can advantageously be registered by means of an detector means. The detector means can also be configured for detecting a translatory movement, and finally errors in the mechanical movement can further be countered by means of a different control means for blocking the mechanical movement.

Preferably there is a communication connection between the control circuits and a protocol will be determined that defines how the communication may occur.

Preferably the driving means is a DC-driven device, e.g. a motor. For controlling the DC-driven device an H-bridge-coupling of electronic switches can advantageously be provided that is controlled by the control circuits thereby enabling the motor to be controlled in both directions and such that each of the control circuits is capable of forcing the motor to halt, irrespective of its direction of running. This is achieved by the current through the DC-driven device passing a pair of switches that are each controlled by each control circuit.

According to a preferred embodiment the control circuits themselves control the electronic switches before a dose is dispensed, and as a further safety measure the invention can by supplemented by a time monitoring device.

According to a preferred embodiment the safety system according to the invention comprises a microprocessor, and the invention also relates to a computer-readable medium containing a program for making a processor carry out safety operations on a system comprising an electrically driven medical delivery device for delivering a dose of medication, wherein the system is configured for preventing delivery of an erroneous dose of the medicament.

The computer-readable medium according to the invention is defined in claim 15.

### BRIEF DESCRIPTION OF THE DRAWINGS

The accompanying drawings illustrate the present invention by way of the embodiments in which:
Fig. 1 illustrates a preferred embodiment of the invention with two detector means and the where the control circuits are in an independent configuration.
Fig. 2 illustrates another embodiment of the invention with a H-bridge and a DC-motor.
Fig. 3 illustrates a prior art problem.
Fig. 4 illustrates an effect of the invention.

### DETAILED DESCRIPTION OF EMBODIMENTS

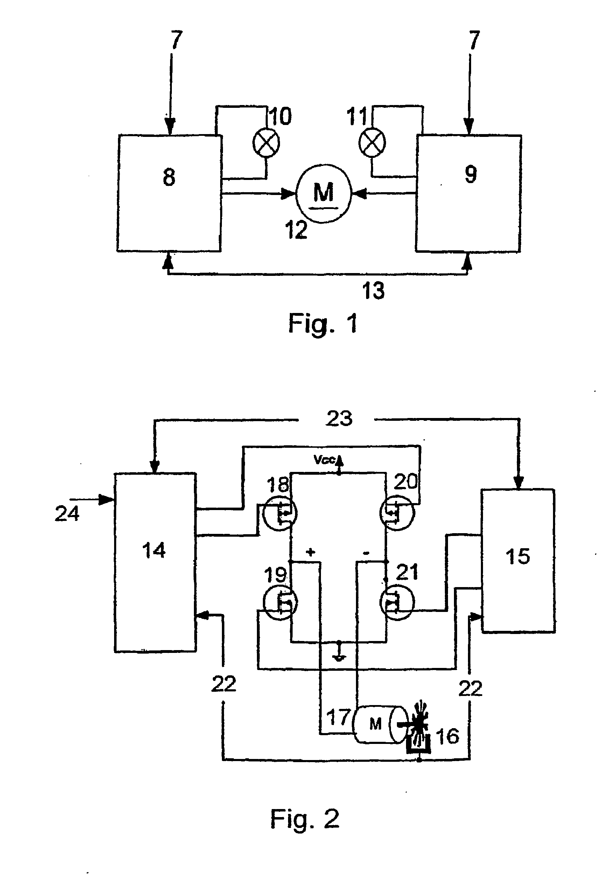

Figure 1 shows an embodiment of the invention according to claim 1, comprising two independent control circuits (8, 9), where the control circuits (8, 9) may both be started from an external signal (7), which further does not necessarily emerge from the same source. However, in preferred embodiments the control circuits may have a common communication (13) link among each other for exchanging information such as status, synchronization i.e.

According to the invention each control circuit (8, 9) comprise detector means (10, 11) being fully independent. That is, they do not depend on each other, nor do they necessarily acquire signals from the same injection related mechanical movement. Detector means (10) of the first control circuit (8) could for example sample directly on the driving means (12), where the detector means (11) of the second control circuit (9) may sample on another injection related mechanical movement along the transmission path that originates in the driving means such as the movement of a piston rod (not showed). Another typical and useful placement of the detector means (10, 11) would be in a gearbox to monitor the rotation of the gearwheels. If for some reasons, a gearwheel does not turn, i.e. due to mechanical breakdown, (moisture) etc. the detector means may alert the matching control circuit to perform any appropriate action.

With a reference to fig. 4 it can be understood that an overdose as explained in connection with fig. 3 can be obviated. If for example the main detector means has ten holes of which two are blocked then the further separate detector means according to the invention will interrupt the delivery when the nominal dose has been delivered. It should be noted that the invention is not solely understood by referring to fig. 4, but a substantial part of the invention consists in realizing the problem explained in connection with figure 3.

Figure 2 illustrates a safety system with two control circuits (14, 15) but where only one detector means (16) monitoring a mechanical movement is shown for the sake of clarity. Driving means (17) are connected to a H-bridge formed by switching means (18, 19, 20, 21) where the switches may be formed by i.e. gates, transistors etc. Detector means (16) are mounted on the driving means (17), which may be a motor, gearbox, piston rod etc., providing a signal (22) by the rotation or translatory movements of the driving means. The detector means signal is fed to the two independent control circuits (14, 15), which in this example are in a master/slave configuration wherein the master circuit (14) receives an external signal (24). The master (14) may remove the supply to the driving means (17) by turning off the switches (18, 20), and the slave (15) may turn off the supply to the driving means (17) by turning off the switches (19, 21). Further, a bi-directional communication possibility exists between the master and the slave circuits (23).

The operation before, during and after injection could be as follows:
1. Each switch in the bridge is tested individually.
2. The detector means are tested.
3. The master communicates the selected dose to the slave.
4. The slave calculates the expected injection time and number of detector means pulses.
5. The slave turns on the appropriate switch in the H-bridge.
6. The master signals to the slave that injection is to begin.
7. The master controls the appropriate switch in the H-bridge to obtain the desired injection speed.
8. During injection the slave monitors time and detector means counts and turns off the relevant switch to disable the driving means if expected time or detector means pulses are exceeded.
9. The master disables the relevant switch when it detects that the desired detector means pulses are reached or if any error, e.g. time-out is encountered.
10. The master inquires the slave about status, which may be information whether the injection ended normally, detector means counts exceeded etc.
11. Each switch in the H-bridge is tested individually.

According to the invention two or more individual detector means are used to give feedback about mechanical movements to the master and slave. They may be mounted on a single part of the mechanical system or as preferred, on separate parts of the mechanical system.

The control circuits may be micro controllers or any similar programmable or any suitable device. Detector means may comprise a tachometer, optical sensor or other detector means and any combination thereof. The driving means may comprise an electrically DC-driven motor, a pump etc. In addition to the said safety system with the ability to turn off the relevant switches, a system is preferred where one or more of the control circuits have control of an additional safety appliance, which exists for blocking the mechanical movement of the driving means. This could be made e.g. in the form of a solenoid mounted with a tap that blocks said movement.

Further, the invention also comprises a computer readable medium containing a program for making a processor carry out safety operations. The program provides two safety routines, each of which depends on an input signal from separate detector means for registering mechanical movement, and each safety routine provides an output signal for controlling driving means for the medical delivery device.

A computer readable medium may in this context be a program storage medium i.e. both physical computer ROM and RAM, removable alike non-removable storage drives, magnetic tape, optical disc, digital video disk (DVD), compact disc (CD or CD-ROM), mini-disc, hard disk, floppy disk, smart card, PCMCIA card, information acquired from data networks e.g. a local area network (LAN), a wide area network (WAN), or any combination thereof, e.g. the Internet, an intranet, an extranet, etc.

That is, a dedicated device with e.g. embedded safety control routines, memory storage and controlling arrangements as well as any remote controllable systems according to the present invention is covered in the claims.

## Claims

1. A safety system for an DC-electrically driven medical delivery device for delivering a dose of a medication, wherein the safety system is configured for preventing delivery of an erroneous dose of the medicament and comprising detector means for detecting a mechanical parameter, said safety system comprising at least two control circuits (8,9) configured for controlling the power supply to driving means (12) for the delivery device, **characterised in that** at least two detector means (10,11) are provided each being arranged for detecting the same mechanical parameter for delivering the dose and for emitting a signal to a respective one of said control circuits, and that said two detector means are arranged in different places along the mechanical transmission path that originates in the delivery device and that each of said detector means is connected to a respective one of said control circuits.

2. A safety system according to claim 1, **characterised in that** the control circuits comprise a master circuit and a slave circuit.

3. A safety system according to claim 1, **characterised in that** the control circuits comprise two separately operating control circuits.

4. A safety system according to claims 1-3, **characterised in that** the output signals from the detector means are processed in the control circuits for fault detection.

5. A safety system according to claims 1-4, **characterised in that** the detector means are configured for detecting a rotational movement.

6. A safety system according to claims 1-5, **characterised in that** the detector means are configured for detecting a translational movement.

7. **A** safety system according to claims 1-6, **characterised in** additional control means for blocking the mechanical movement.

8. A safety system according to claim 2 or 3, **characterised in that** a communication connection is provided between the control circuits.

9. A safety system according to claims 1-8, **characterised in that** the driving means comprise a DC-driven device.

10. A safety system according to claim 9, **characterised in that** the DCdriven device is connected to the centres of a H-bridge coupling of electronic switches that are connected to a power supply source and that are controlled by the control circuits.

11. A safety system according to claim 10 , **characterised in that** the electronic switches are connected to the control circuits such that the current through the DC-driven device passes a pair of switches that are each controlled by each their control circuits.

12. A safety system according to claim 11, **characterised in that** the control circuits are configured for testing said electronic switches prior to delivery of the dose.

13. A safety system according to claims 1-12, **characterised in that** at least the one control circuit comprises a time monitoring device.

14. A safety system according to claims 1-13, **characterised in** comprising a microprocessor.

15. A computer readable medium containing a program for making a processor carry out safety operations on a system comprising a DC-electrically driven medical delivery device for delivering a dose of a medication, wherein the system is configured for preventing delivery of an erroneous dose of the medicament and comprises at least two control circuits, **characterised in** said safety operations comprising:
providing two safety routines, each of which depends on an input signal from one of two respective detector means for registering mechanical movement, said two detector means being arranged in different places along the mechanical transmission path that originates in the delivery device and that each of said detector means are connected to a respective one of the at least two control circuits; and wherein each safety routine provides an output signal for controlling driving means for the medical delivery device.

## Patentansprüche

1. Sicherheitssystem für eine mit Gleichstrom elektrisch angetriebene medizinische Abgabevorrichtung zum Abgeben einer Dosis eines Medikaments, wobei das Sicherheitssystem zum Verhindern der Abgabe einer fehlerhaften Dosis des Medikaments konfiguriert ist und ein Erfassungsmittel zum Erfassen eines mechanischen Parameters umfasst, wobei das System mindestens zwei Steuerschaltungen (8, 9) aufweist, die zum Steuern der Stromzufuhr zum Antriebsmittel (12) für die Abgabevorrichtung konfiguriert sind, **dadurch gekennzeichnet, dass** mindestens zwei Erfassungsmittel (10, 11) bereitgestellt sind, wobei jedes zum Erfassen des gleichen mechanischen Parameters zur Abgabe der Dosis und zum Emittieren eines Signals an eine jeweilige der Steuerschaltungen angeordnet sind, und dass die zwei Erfassungsmittel an unterschiedlichen Stellen entlang des mechanischen Übertragungswegs angeordnet sind, der in der Abgabevorrichtung entsteht, und dass jedes der Erfassungsmittel an eine jeweilige der Steuerschaltungen angeschlossen ist.

2. Sicherheitssystem nach Anspruch 1, **dadurch gekennzeichnet, dass** die Steuerschaltungen eine Masterschaltung und eine Slaveschaltung umfassen.

3. Sicherheitssystem nach Anspruch 1, **dadurch gekennzeichnet, dass** die Steuerschaltungen zwei separat arbeitende Steuerschaltungen umfassen.

4. Sicherheitssystem nach Anspruch 1-3, **dadurch gekennzeichnet, dass** die Ausgangssignale von den Erfassungsmitteln in den Steuerschaltungen auf Fehlererfassung verarbeitet werden.

5. Sicherheitssystem nach Anspruch 1-4, **dadurch gekennzeichnet, dass** die Erfassungsmittel zum Erfassen einer Drehbewegung konfiguriert sind.

6. Sicherheitssystem nach Anspruch 1-5, **dadurch gekennzeichnet, dass** die Erfassungsmittel zum Erfassen einer Verschiebungsbewegung konfiguriert sind.

7. Sicherheitssystem nach Anspruch 1-6, **gekennzeichnet durch** zusätzliche Steuermittel zum Blockieren der mechanischen Bewegung.

8. Sicherheitssystem nach Anspruch 2 oder 3, **dadurch gekennzeichnet, dass** eine Kommunikationsverbindung zwischen den Steuerschaltungen bereitgestellt ist.

9. Sicherheitssystem nach Anspruch 1-8, **dadurch gekennzeichnet, dass** das Antriebsmittel eine mit Gleichstrom angetriebene Vorrichtung umfasst.

10. Sicherheitssystem nach Anspruch 9, **dadurch gekennzeichnet, dass** die mit Gleichstrom angetriebene Vorrichtung an die Zentren einer H-Brücke angeschlossen ist, die elektronische Schalter koppelt, die an eine Stromquelle angeschlossen sind und durch die Steuerschaltungen gesteuert werden.

11. Sicherheitssystem nach Anspruch 10, **dadurch gekennzeichnet, dass** die elektronischen Schalter an die Steuerschaltungen derart angeschlossen sind, dass der Strom durch die mit Gleichstrom angetriebene Vorrichtung ein Paar Schalter durchströmt, die jeder durch jede ihre Steuerschaltungen gesteuert werden.

12. Sicherheitssystem nach Anspruch 11, **dadurch gekennzeichnet, dass** die Steuerschaltungen zum Testen der elektronischen Schalter vor der Abgabe der Dosis konfiguriert sind.

13. Sicherheitssystem nach Anspruch 1-12, **dadurch gekennzeichnet, dass** mindestens die eine Steuerschaltung eine Zeitüberwachungsvorrichtung umfasst.

14. Sicherheitssystem nach Anspruch 1-13, **dadurch gekennzeichnet, dass** es einen Mikrocomputer umfasst.

15. Computerlesbares Medium, enthaltend ein Programm zum Herstellen eines Prozessors, der Sicherheitsvorgänge an einem System, umfassend eine mit Gleichstrom angetriebene medizinische Abgabevorrichtung zur Abgabe einer Dosis eines Medikaments ausführt, wobei das System zum Verhindern einer Abgabe einer fehlerhaften Dosis des Medikaments konfiguriert ist und mindestens zwei Steuerschaltungen umfasst, **gekennzeichnet durch** die zwei Sicherheitsvorgänge, umfassend:
Bereitstellen von zwei Sicherheitsroutinen, wobei jede davon von einem Eingangssignal von einer der zwei jeweiligen Erfassungsmittel zum Registrieren einer mechanischen Bewegung abhängt, wobei die zwei Erfassungsmittel an unterschiedlichen Stellen entlang des mechanischen Übertragungswegs angeordnet sind, der in der Abgabevorrichtung entsteht, und dass jedes der Erfassungsmittel mit einer jeweiligen der mindestens zwei Steuerschaltungen verbunden ist; und wobei jede Sicherheitsroutine ein Ausgangssignal zum Steuern des Antriebsmittels für die medizinische Abgabevorrichtung bereitstellt.

## Revendications

1. Un système de sécurité pour un dispositif de délivrance médicale à entraînement électrique en courant continu pour délivrer une dose d'un médicament, dans lequel le système de sécurité est configuré pour empêcher la délivrance d'une dose erronée du médicament et comprenant des moyens détecteurs pour détecter un paramètre mécanique, ledit système de sécurité comprenant au moins deux circuits de contrôle (8, 9) configurés pour contrôler l'alimentation en énergie des moyens d'entraînement (12) pour le dispositif de délivrance, **caractérisé en ce qu'**il est prévu au moins deux moyens détecteurs (10, 11), chacun étant configuré pour détecter le même paramètre mécanique pour délivrer la dose et pour émettre un signal vers l'un respectif desdits circuits de contrôle, et **en ce que** lesdits deux moyens détecteurs sont configurés en des emplacements différents le long du trajet de transmission mécanique qui part du dispositif de délivrance et **en ce que** chacun desdits moyens détecteurs est relié à l'un respectif desdits circuits de contrôle.

2. Un système de sécurité selon la revendication 1, **caractérisé en ce que** les circuits de contrôle comprennent un circuit maître et un circuit esclave.

3. Un système de sécurité selon la revendication 1, **caractérisé en ce que** les circuits de contrôle comprennent deux circuits de contrôle opérant de manière séparée.

4. Un système de sécurité selon les revendications 1 à 3, **caractérisé en ce que** les signaux de sortie provenant des moyens détecteurs sont traités dans les circuits de contrôle pour détecter des anomalies.

5. Un système de sécurité selon les revendications 1 à 4, **caractérisé en ce que** les moyens détecteurs sont configurés pour détecter un mouvement de rotation.

6. Un système de sécurité selon les revendications 1 à 5, **caractérisé en ce que** les moyens détecteurs sont configurés pour détecter un mouvement de translation.

7. Un système de sécurité selon les revendications 1 à 6, **caractérisé par** des moyens de contrôle additionnels pour bloquer le mouvement mécanique.

8. Un système de sécurité selon la revendication 2 ou 3, **caractérisé en ce qu'**il est prévu un lien de communication entre les circuits de contrôle.

9. Un système de sécurité selon les revendications 1 à 8, **caractérisé en ce que** les moyens d'entraînement comprennent un dispositif entraîné en courant continu.

10. Un système de sécurité selon la revendication 9, **caractérisé en ce que** le dispositif entraîné en courant continu est relié aux points milieux d'un couplage à pont en H de commutateurs électroniques qui sont reliés à une source d'alimentation d'énergie et qui sont contrôlés par les circuits de contrôle.

11. Un système de sécurité selon la revendication 10, **caractérisé en ce que** les commutateurs électroniques sont reliés aux circuits de contrôle de telle sorte que le courant traversant le dispositif entraîné en courant continu passe par une paire de commutateurs qui sont chacun contrôlés par chacun de leurs circuits de contrôle.

12. Un système de sécurité selon la revendication 11, **caractérisé en ce que** les circuits de contrôle sont configurés pour tester lesdits commutateurs électroniques avant délivrance de la dose.

13. Un système de sécurité selon les revendications 1 à 12, **caractérisé en ce qu'**au moins l'un des circuits de contrôle comprend un dispositif de surveillance du temps.

14. Un système de sécurité selon les revendications 1 à 13, **caractérisé en ce qu'**il comprend un microprocesseur.

15. Un support lisible par ordinateur contenant un programme pour permettre à un processeur d'exécuter des instructions de sécurité sur un système comprenant un dispositif de délivrance médicale entraîné électriquement en courant continu pour délivrer une dose d'un médicament, dans lequel le système est configuré pour empêcher la délivrance d'une dose erronée du médicament et comprend au moins deux circuits de contrôle, **caractérisé en ce que** lesdites opérations de sécurité comprennent: prévoir deux routines de sécurité, chacune d'entre elles dépendant d'un signal d'entrée provenant de l'un de deux moyens détecteurs respectifs pour enregistrer un mouvement mécanique, lesdits deux moyens détecteurs étant configurés en des emplacements différents le long du trajet de transmission mécanique qui part du dispositif de délivrance et **en ce que** chacun desdits moyens détecteurs est relié à l'un respectif des au moins deux circuits de contrôle ; et dans lequel chaque routine de sécurité produit un signal de sortie pour contrôler les moyens d'entraînement pour le dispositif de délivrance médicale.
